# EUROPEAN PATENT SPECIFICATION

(11) **EP 0 675 866 B1**
(45) Date of publication and mention of the grant of the patent: **04.06.1997**
(21) Application number: 94902773.4
(22) Date of filing: 09.12.1993
(51) Int. Cl.: C07C 31/36, C07C 29/66

(54) **PROCESS FOR THE PRODUCTION OF DICHLOROHYDRIN**
VERFAHREN ZUR HERSTELLUNG VON DICHLORHYDRIN
PROCEDE DE PRODUCTION DE DICHLORHYDRINE

(30) Priority: 11.12.1992 US 989621
(43) Date of publication of application: 11.10.1995
(73) Proprietor: SHELL INTERNATIONALE RESEARCH MAATSCHAPPIJ B.V., 2596 HR Den Haag (NL)
(72) Inventor: MAAS, Wilfridus, Petrus, Maria, NL-1031 CM Amsterdam (NL); PETRUS, Leonardus, NL-3196 KK Rotterdam (NL); JUNE, Ronald, Ken, Houston, TX 77079 (US); NISBET, Timothy, Michael, NL-1031 CM Amsterdam (NL)
(86) International application number: EP9303546
(87) International publication number: WO9413611

(56) References cited:
- EP-A- 0 228 118
- EP-A- 0 359 331
- EP-A- 0 537 846

## Description

The present invention relates to a process for the production of dichlorohydrin. The term dichlorohydrin as employed herein covers both isomers 1,2-dichloro-3-hydroxypropane and 1,3-dichloro-2-hydroxypropane.

It is known to prepare dichlorohydrin by reacting an allyl chloride feed with water and chlorine in a dilute aqueous phase. Allyl chloride is commercially prepared by the high-temperature chlorination of propene. The main use of dichlorohydrin is for preparing epichlorohydrin (1,2-epoxy-3-chloropropane), by dehydrochlorination in the presence of a base. The subject reactions may be carried out batchwise, semi-continuously or continuously.

It is also known, that the voluminous aqueous effluent emerging from the epichlorohydrin plant contains appreciable amounts of other chlorinated organic compounds (Extractable Organic Chlorine, EOCl, up to about 100 mg Cl/l), mainly chloroaliphatic ethers and chloroaliphatic alkanes. Since these chlorinated organic by-products are toxic, their concentration in the waste water should be reduced as much as possible before the waste water is fit for being passed to receiving bodies such as rivers and lakes. Since the removal of the chlorinated organic by-products from the epichlorohydrin plant effluent by conventional methods, such as fractional distillation is very expensive, there exists a need for an alternative method for reducing their level.

The present applicant's EP-A-0 359 331 is directed to a method of reducing the level of chlorinated organic by-products in the above-described reaction effluent by extracting the aqueous product of the reaction of allyl chloride with water and chlorine, with a water-immiscible solvent having an atmospheric boiling point of from 40 °C to 105 °C. The present applicant's EP-A-0 537 846 discloses a similar method wherein the extracting water-immiscible solvent is 1,2,3-trichloropropane.

The present invention now presents a different approach to reducing the level of chlorinated organic by-products in the above-described reaction effluent.

As indicated above, these chlorinated organic by-products are mainly chloroaliphatic ethers and chloroaliphatic alkanes. These are already present in the conventionally-prepared dichlorohydrin, and they are carried to the effluent of the epichlorohydrin plant. In particular, they were found to be mainly chloroaliphatic ethers having 9 and 12 carbon atoms (C₉ and C₁₂), the level of the C₁₅ chloroaliphatic ethers being much lower.

The present inventors investigated the source of these chlorinated organic by-products. Theoretically, they would be expected to originate from oligomerisation reactions of reactive intermediates formed from allyl chloride and chlorine with water and/or dichlorohydrin. However, the present inventors found their main source to be hexadienes (C₆H₁₀ isomers, in particular 1,5-hexadiene, 1,4-hexadiene and 2-methyl-1,4-pentadiene), which are by-products of the high temperature chlorination of propene to allyl chloride. When present in the allyl chloride feed to the above-described reaction with water and chlorine to form dichlorohydrin, this hexadiene also reacts with chlorine, water and dichlorohydrin to form the chloroaliphatic ethers found in the reaction effluent.

Commercial allyl chloride is at least 97.5 wt% pure and contains hexadiene as an impurity (Ullman's Encyclopaedia of Industrial Chemistry, 5th Edition, vol. A 1985, page 431). Normally, 0.3-1.0 wt%, of hexadiene is present. It has now been found that when an allyl chloride feed containing less than 0.30 wt% of hexadiene is used with water and chlorine for the production of dichlorohydrin, the product contains much less of the undesirable chlorinated organic by-products, which substantially reduces the costs of purifying the aqueous effluent leaving the plant.

The present invention therefore is directed to a process for the preparation of dichlorohydrin by reacting an allyl chloride feed with water and chlorine, characterized in that an allyl chloride feed is used which contains less than 0.30 wt% of hexadiene.

Preferably, the allyl chloride feed contains less than 0.20 wt%, more preferably less than 0.10 wt% of hexadiene.

It will be appreciated, that ideally the allyl chloride feed according to the invention will contain no (0) hexadiene, and that practical and economical considerations will play an important role in determining the actual reduction employed.

Since commercially available allyl chloride usually contains at least 0.3 wt% of hexadiene, the use of an allyl chloride feed containing less than 0.30 wt% of hexadiene in the process according to the invention will generally involve a step of removing excess hexadiene from such commercially available allyl chloride. This may be performed by selective distillation of the hexadiene, or alternatively by reacting the hexadiene to form compounds which are more easily removable. However, it should also be possible to devise methods for directly producing allyl chloride having a low content of hexadiene. Thus, the reactor according to the present applicant's EP-B-0 107 215 is expected to effect a more efficient chlorination of propene to allyl chloride and less production of by-products, including hexadiene. Furthermore, beside the high temperature chlorination of propene, different reactions for the production of allyl chloride are also feasible, which will also produce allyl chloride having a lower content of hexadiene.

Selective distillation is a preferred method of removing hexadiene from commercially available allyl chloride feed. The crude allyl chloride, as produced by the chlorination of propene, always has to be purified to a content of at least 97.5 wt% of allyl chloride before being used as a feed for the production of dichlorohydrin, and this purification is usually done by distillation. However, considerably more intensive distillation is required in order to separate the hexadienes from the allyl chloride, in particular when the hexadienes are predominantly present as their low-boiling isomers, as is generally the case in crude, untreated allyl chloride. The boiling point at atmospheric pressure of allyl chloride is 45 °C and that of 2-methyl-1,4-pentadiene, 1-5-hexadiene and 1,4-hexadiene is 55 °C, 59,5 °C and 65 °C respectively.

A considerably lower strain is placed on the distillation step by first isomerising the lower-boiling hexadienes which form the greater part of the hexadienes in untreated allyl chloride to the higher boiling hexadiene isomers. In particular, the higher boiling 1,3-hexadiene and 2,4-hexadiene, having a boiling point at atmospheric pressure of 73 °C and 80 °C respectively, are not normally present in untreated allyl chloride.

The isomerisation step can be performed by using known acid catalysts, preferably solid acid catalysts, such as ion exchange resins. Strong acid catalysts are preferred. Particularly preferred are the macroporous ion exchange resins, such as Amberlist 15 (H⁺), trade mark of Rohm & Haas. Homogenous acid catalysts can also be used in principle. The isomerisation reaction can be performed continuously, or batchwise.

The effective amount of macroporous ion exchange catalyst for the continuous treatment of a given flow of hexadiene-containing allyl chloride will depend on the reaction temperature. The temperature range within which the ion exchange resin can effectively be used is 0 - 150 °C and preferably 20 - 120 °C. The catalyst amount required to effectively treat 1 kg/h of hexadiene-containing allyl chloride is 0.1 - 100 equivalents of H⁺ (typically corresponding to 0.05 - 50 L of catalyst bed volume) and preferably 0.2 - 50 equivalents. The lower amounts of catalyst are typically used at the higher reaction temperatures and vice versa. The pressure is preferably selected such that the system is operated in the liquid phase, by keeping it (slightly) above the vapour pressure, at the reaction temperature used, of the allyl chloride.

The present applicants found, that in the presence of an acid catalyst this isomerisation of the hexadienes can be performed selectively, without concurrently modifying the allyl chloride (to 1-chloro-propene) in any appreciable amount.

An alternative to the step of isomerising the low-boiling hexadienes in allyl chloride to higher-boiling hexadienes prior to distillation, is the step of converting the hexadienes by hydrochlorination to chlorohexenes and dichlorohexanes. The boiling points at atmospheric pressure of chlorohexenes and of dichlorohexanes being in the ranges of 110 - 140 °C and 170 - 220 °C respectively, their separation in a further distillation step from allyl chloride is very simple. When the product of the hydrochlorination step contains predominantly chlorohexanes with only low levels of chlorohexenes, no special distillation step is strictly required following the hydrochlorination step.

The present applicants found, that in the presence of a Lewis acid this hydrochlorination of the hexadienes can be performed selectively, without concurrently modifying the allyl chloride (to 1,2-dichloropropane) in any appreciable amount.

Examples of suitable Lewis acids are FeCl₃, ReCl₅, ZnCl₂ and SbCl₅. A preferred Lewis acid catalyst was found to be MoCl_{5.} In order to treat 1 kg/h of allyl chloride feed, an amount of the Lewis acid generally ranging from 0.01 to 100 mmol, preferably from 0.1 to 10 mmole, is effective. The catalyst can be immobilised in a manner known in the art, for example on graphite or on a sulphonated polystyrene resin.

The concentration of HCl used to hydrochlorinate the hexadienes is preferably kept within the range of 0.1 to 5.0 wt%, based on the total amount of the allyl chloride containing feed, since much higher concentrations of HCl will tend to hydrochlorinate the allyl chloride as well. The pressure is preferably selected such that the system is operated in the liquid phase, by keeping it (slightly) above the vapour pressure, at the reaction temperature used, of the allyl chloride.

The following Examples will illustrate the invention.

### EXAMPLE 1

In a reactor of 1.7 1 content, 1 1 of water was charged at ambient temperature. To this 0.5 mol of allyl chloride and 0.5 mol of chlorine were added continuously at constant rates over a period of 2 hours. During the reaction, temperature increased to 40 °C. Shortly after completion of the addition the reaction stopped. Subsequently, 16 g of n-hexane were added to the aqueous reaction mixture. Following intensively stirring the mixture was allowed to settle, thereby separating into the aqueous and organic phase.

The organic phase was analysed for chloroaliphatic ethers (C₉+C₁₂+C₁₅), using gas chromatography, equipped with a chlorine-selective detector having a linear response (HALL).

Table 1 presents the results of Experiment 1.1 and Comparative Experiment 1.2, wherein the > 99% allyl chloride feed used respectively contained 0.032 wt% and 0.5 wt% of hexadiene (Hex/AC). Therein, the chloroaliphatic ethers contents are expressed in mg Cl per litre of the aqueous reaction mixture.

**TABLE 1**

| Exp. No. | Hex./AC wt% | Chloroaliphatic ethers, mg Cl/l of reaction mixture |
|---|---|---|
| 1.1 | 0.032 | 22.0 |
| 1.2 | 0.500 | 171.3 |

From these results it is apparent, that the 94% reduction (from 0.5 wt% to 0.032 wt%) of hexadiene content in the allyl chloride feed resulted in a 87% reduction (from 171.3 to 22.0 Cl/l) of the chloroaliphatic ethers content in the reaction effluent.

### EXAMPLE 2

In five laboratory-scale experiments in an OLDERSHAW-type distillation column, using mass reflux over distillate ratios (R/D ratio) of 0.3, 1, 2, 4 and 6 respectively, a continuous stream of crude allyl chloride feed was subjected to distillation over 120 trays. The feed tray was the 60th, the feed temperature 41-45 °C, the bottom temperature 91-95 °C and the Mass Top Flow Rate 72-75% of the feed.

The composition of the allyl chloride feed, as determined by gas chromatography, before distillation and in the top product of the distillation column, is presented in Table 1.

**TABLE 2**

| component | wt% in crude feed | wt% in top distillate stream, when using R/D ratio | | | | |
|---|---|---|---|---|---|---|
| | | 0.3 | 1 | 2 | 4 | 6 |
| Allyl chloride | 75.22 | 98.92 | 99.09 | 99.29 | 99.25 | 99.29 |
| 1,5-hexadiene | 0.33 | 0.33 | 0.26 | 0.20 | 0.03 | 0.01 |
| Total hexadiene | 0.39 | 0.41 | 0.33 | 0.26 | 0.06 | 0.02 |

From these results it appears, that much less intensive distillation is needed in order to purify the crude allyl chloride to above 98-99 wt%, than is needed in order to reduce the hexadiene content to below 0.20 wt%.

### EXAMPLE 3

A continuous stream of commercially available purified (98.2 wt%) allyl chloride, containing a total of 0.4 wt% hexadienes 90% of which were 1,5-hexadiene, was fed to a 140 ml pressurised and thermostatically controlled reactor, containing a fixed bed of 86 gram (dry) Amberlite 15 (H⁺) ion exchanger. The feed flow was 70 ml/h (LHSV 0,5), the pressure 400 kPa and the temperature 90 °C.

When steady state was reached, the outflow stream from the reactor also contained a total of 0.4 wt% hexadienes but only 12.5% of this (0.05 wt%) was 1,5-hexadiene and about 75% was 2,4-hexadiene.

This outflow stream was subjected to distillation in an OLDERSHAW-type distillation column as in Example 2, the differences being that the mass reflux over distillation ratio (R/D ratio) was 0.8, the number of trays 40 (the feed tray being the 20th) and the mass top flow rate 90% of the feed. The top distillate stream contained 0.04 wt% of total hexadiene, most of which was 1,5-hexadiene.

### EXAMPLE 4

A continuous stream of crude (80 wt%) allyl chloride, containing a total of 0.4 wt% hexadienes 90% of which were 1,5-hexadiene, was fed to a 140 ml pressurised and thermostatically controlled reactor, containing a fixed bed of 86 gram (dry) Amberlite 15 (H⁺) ion exchanger. The feed flow was 30 ml/h (LHSV 0.2), the pressure 400 kPa and the temperature 70 °C.

When steady state was reached, the outflow stream from the reactor also contained a total of 0.4 wt% hexadienes but only 7.5% of this (0.03 wt%) was 1,5-hexadiene and about 85% was 2,4-hexadiene.

This outflow stream was subjected to distillation in an OLDERSHAW-type distillation column as in Example 2, the differences being that the mass reflux over distillation ratio (R/D ratio) was 1.8, the number of trays 60 (the feed tray being the 20th from the top) and the mass top flow rate 99% of the feed. The top distillate stream contained 0.02 wt% of total hexadiene, most of which was 1,5-hexadiene.

### EXAMPLE 5

Three hydrochlorination experiments (5.1-5.3) using MoCl₅ as catalyst were carried out batchwise in the liquid phase, in a well-mixed and jacketed 750 ml glass reactor at a pressure of 220, 230 and 300 kPa respectively and over a range of reaction temperatures (18, 38 and 68 °C respectively). Prior to the reaction, the reactor was purged with nitrogen and the catalyst was also handled and transferred to the reactor under nitrogen. Allyl chloride was charged to the reactor in a total amount of 270, 315 and 320 g respectively together with 2.2, 1.8 and 0.9 wt% respectively of HCl and 0.33, 0.39 and 0.41 wt% respectively of MoCl₅, based on the amount of the feed. When the reactor contents were at the desired temperature, 1,5-hexadiene was added in an amount of 0.46, 0.46 and 0.41 wt% respectively. After 40, 20 and 20 minutes respectively the reaction was terminated and the contents of the reactor were analysed by gas chromatography. The results are summarised in Table 3.

**TABLE 3**

| Exp. No. | Product analysis, wt% | | |
|---|---|---|---|
| | 1,5-hexadiene | chlorohexenes | dichlorohexanes |
| 5.1 | 0.01 | 0.38 | 0.32 |
| 5.2 | <0.01 | 0.06 | 0.72 |
| 5.3 | 0.02 | 0.18 | 0.51 |

From these results it appears, that in the above three experiments the reaction resulted in a conversion of 98, >98 and 95% respectively of the initially present 1,5-hexadiene to chlorohexenes and dichlorohexanes.

## Claims

1. A process for the preparation of dichlorohydrin by reacting an allyl chloride feed with water and chlorine, characterized in that an allyl chloride feed is used which contains less than 0.30 wt% of hexadiene.

2. A process according to claim 1, characterized in that the hexadiene content in the allyl chloride feed used is less than 0.20 wt%.

3. A process according to claim 2, characterized in that the hexadiene content in the allyl chloride feed used is less than 0.10 wt%.

4. A process according to any one of claims 1-3, characterized in that the allyl chloride feed used is prepared by removing hexadiene from an allyl chloride feed which contains at least 0.30 wt% of hexadiene.

5. A process according to claim 4, characterized in that the removal of hexadiene is performed by distillation.

6. A process according to claim 4 or 5, characterized in that the removal of hexadiene is performed by reacting the hexadiene, to form an easily removable compound.

7. A process according to claim 6, characterized in that the reaction is an isomerisation reaction whereby low-boiling isomers of hexadiene are converted to higher boiling hexadienes in the presence of an acid catalyst.

8. A process according to claim 7, characterized in that the acid catalyst is a macroporous strongly acid ion exchange resin.

9. A process according to claim 6, characterized in that the reaction is a hydrochlorination reaction, whereby hexadienes are converted to chlorohexenes and dichlorohexanes in the presence of a Lewis acid catalyst.

10. A process according to claim 9, characterized in that the Lewis acid catalyst is MoCl₅.

## Patentansprüche

1. Verfahren zur Herstellung von Dichlorhydrin durch Umsetzung eines Allylchlorid-Einsatzstoffs mit Wasser und Chlor, dadurch gekennzeichnet, daß man einen Allylchlorid-Einsatzstoff verwendet, der weniger als 0,30 Gew.-% Hexadien enthält.

2. Verfahren nach Anspruch 1, dadurch gekennzeichnet, daß man einen Allylchlorid-Einsatzstoff verwendet, der weniger als 0,20 Gew.-% Hexadien enthält.

3. Verfahren nach Anspruch 2, dadurch gekennzeichnet, daß man einen Allylchlorid-Einsatzstoff verwendet, der weniger als 0,10 Gew.-% Hexadien enthält.

4. Verfahren nach einem der Ansprüche 1-3, dadurch gekennzeichnet, daß man den verwendeten Allylchlorid-Einsatzstoff durch Entfernung von Hexadien aus einem Allylchlorid-Einsatzstoff, der mindestens 0,30 Gew.-% Hexadien enthält, herstellt.

5. Verfahren nach Anspruch 4, dadurch gekennzeichnet, daß man das Hexadien durch Destillation entfernt.

6. Verfahren nach Anspruch 4 oder 5, dadurch gekennzeichnet, daß man das Hexadien durch Umsetzung zu einer leicht zu entfernenden Verbindung abtrennt.

7. Verfahren nach Anspruch 6, dadurch gekennzeichnet, daß man als Umsetzung eine Isomerisierungsreaktion durchführt, durch die niedrig siedende Hexadien-Isomere in Gegenwart eines sauren Katalysators in höher siedende Hexadiene umgewandelt werden.

8. Verfahren nach Anspruch 7, dadurch gekennzeichnet, daß man als sauren Katalysator ein makroporöses, stark saures Ionenaustauscherharz einsetzt.

9. Verfahren nach Anspruch 6, dadurch gekennzeichnet, daß man als Umsetzung eine Hydrochlorierungsreaktion durchführt, durch die Hexadiene in Gegenwart eines Lewissäure-Katalysators in Chlorhexene und Dichlorhexane umgewandelt werden.

10. Verfahren nach Anspruch 9, dadurch gekennzeichnet, daß man als Lewissäure-Katalysator MoCl₅ einsetzt.

## Revendications

1. Procédé de préparation de la dichlorhydrine par la réaction d'une charge de chlorure d'allyle avec l'eau et le chlore, caractérisé en ce que l'on utilise une charge de chlorure d'allyle qui contient moins de 0,30% en poids d'hexadiène.

2. Procédé suivant la revendication 1, caractérisé en ce que la teneur en hexadiène de la charge de chlorure d'allyle utilisée est inférieure à 0,20% en poids.

3. Procédé suivant la revendication 2, caractérisé en ce que la teneur en hexadiène de la charge de chlorure d'allyle utilisée est inférieure à 0,10% en poids.

4. Procédé suivant l'une quelconque des revendications 1 à 3, caractérisé en ce que l'on prépare la charge de chlorure d'allyle utilisée en éliminant l'hexadiène d'une charge de chlorure d'allyle qui contient au moins 0,30% en poids d'hexadiène.

5. Procédé suivant la revendication 4, caractérisé en ce que l'élimination de l'hexadiène se réalise par distillation.

6. Procédé suivant la revendication 4 ou 5, caractérisé en ce que l'on réalise l'élimination de l'hexadiène en faisant réagir l'hexadiène pour former un composé facile à éliminer.

7. Procédé suivant la revendication 6, caractérisé en ce que la réaction est une réaction d'isomérisation au cours de laquelle des isomères de l'hexadiène à bas point d'ébullition sont convertis en hexadiènes à point d'ébullition supérieur en présence d'un catalyseur acide.

8. Procédé suivant la revendication 7, caractérisé en ce que le catalyseur acide est une résine échangeuse d'ions fortement acide et macroporeuse.

9. Procédé suivant la revendication 6, caractérisé en ce que la réaction est une réaction d'hydrochloruration, conformément à laquelle des hexadiènes sont convertis en chlorhexènes et en dichlorhexanes en présence d'un catalyseur à acide de Lewis.

10. Procédé suivant la revendication 9, caractérisé en ce que le catalyseur à acide de Lewis est le MoCl₅.
